# EUROPEAN PATENT SPECIFICATION

(11) **EP 2 983 671 B1**
(45) Date of publication and mention of the grant of the patent: **24.10.2018**
(21) Application number: 14715933.9
(22) Date of filing: 09.04.2014
(51) Int. Cl.: A61K 31/567, A61P 15/00

(54) **PROGESTERONE RECEPTOR ANTAGONIST DOSAGE FORM**
DOSIERUNGSFORM FÜR EINEN PROGESTERON-REZEPTORANTAGONISTEN
FORME POSOLOGIQUE DE L'ANTAGONISTE DU RÉCEPTEUR DE LA PROGESTÉRONE

(30) Priority: 11.04.2013 EP 13163417
(43) Date of publication of application: 17.02.2016
(73) Proprietor: Bayer Pharma Aktiengesellschaft, 13353 Berlin (DE)
(72) Inventor: SCHÜTT, Barbara, 12249 Berlin (DE); SCHULTZE-MOSGAU, Marcus-Hillert, 16548 Glienicke/Nordbahn (DE); KAISER, Andreas, 12209 Berlin (DE)
(74) Representative: BIP Patents
(86) International application number: PCT/EP2014/057101
(87) International publication number: WO 2014/166971

(56) References cited:
- US-A1- 2012 094 969
- US-A1- 2012 258 941
- MÖLLER ET AL: "Investigational developments for the treatment of progesterone dependent diseases", EXPERT. OPINION INVEST. DRUGS, vol. 17, no. 4, 2008, - 2008, pages 469-479, XP002723960,

## Description

The invention is directed to a pharmaceutical composition comprising a progesterone receptor antagonist namely (11β,17β)-17-Hydroxy-11-[4-(methylsulphonyl)phenyl]-17-(pentafluoroethyl)estra-4,9-dien-3-one at the dosage of about 0.5 to 5 mg and more particularly 2 mg. This compound is for the use of the novel pharmaceutical composition for treatment of and/or prophylaxis of gynaecological diseases, such as fibroids of the uterus (myomas, uterine leiomyoma), endometriosis or excessive menstrual bleeds, and method for obtaining such composition and oral dosage form.

### Background

Uterine leiomyomas (also called fibroids or myomas) are common benign tumors of the myometrium, which are reported to occur in approximately 30-40 % of all women of reproductive age. They may remain asymptomatic, or cause bleeding abnormalities and/or bulk-related symptoms depending on their number, size and location. Various medications are used for symptom-oriented therapy in minor disease (e.g. combined oral contraceptives, progestogens, iron supplements). For short-term therapy and/or as a precursor to surgery, gonadotropin-releasing hormone agonists represent the most effective medical treatment. However, their use is restricted to 6 months due to hypoestrogenic side effects. For definite treatment of symptomatic leiomyomas, therapeutic options are mainly surgical so far.
Various studies suggested steroid-dependence of fibroids growth in which progesterone has a critical role. This is supported by the fact, that progesterone receptor (PR) antagonists - like mifepristone (RU 486) - have been shown to decrease the size of fibroids and related symptoms. Therefore, PR antagonists might offer a promising therapeutic alternative meeting the need for medical long-term treatment of symptomatic fibroids with an orally effective agent lacking clinically relevant side effects. Mifepristone (RU 486) was disclosed in EP57115. Additional competitive progesterone receptor modulators are shown below. 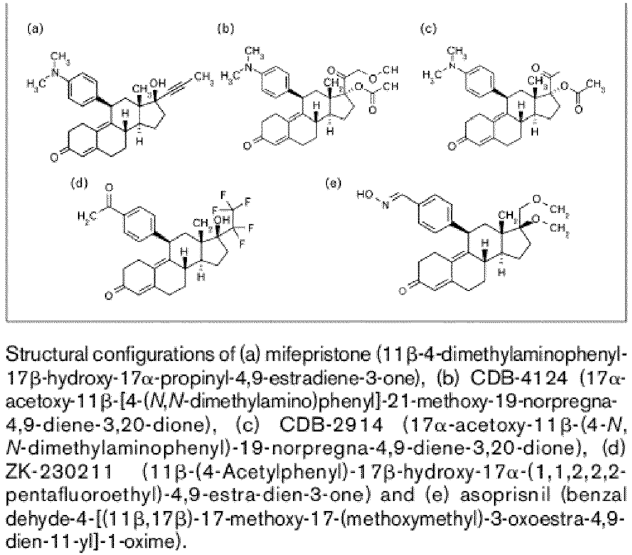 from Spitz et al. Current Opinion in Obstetrics and Gynecology, 2009, 21:318-324.
All these compounds as listed above are effective in the treatment of uterine fibroids where that are associated with a reduction in pain, bleeding and improvement in quality of life and decrease in fibroid size. Long-term treatments are associated with endometrial thickening on ultrasound and histological changes in the endometrium. The endometrial change such as endometrial thickening seems to be connected to cystic glandular dilatation (Spitz et al. Current Opinion in Obstetrics and Gynecology, 2009, 21:318-324.).

Progesterone receptor antagonists with a fluorinated 17a-side chain were published in WO 98/34947 and Fuhrmann et al., J. Med. Chem. 43, 5010 - 5016 (2000).

In PEARL I and PEARL II trial (N Engl J Med. 2012;366:409-420) women with excessive uterine bleeding due to the presence of fibroids were randomized to ulipristal acetate (5 mg vs. 10 mg orally once daily) vs. placebo or intramuscular injections of leuprolide acetate for up to 13 weeks.

To conclude, endometrium changes were observed during treatment with most of the cited above progesterone receptor antagonists.

It was surprisingly found that (11β,17β)-17-Hydroxy-11-[4-(methylsulphonyl)phenyl]-17-(pentafluoroethyl)estra-4,9-dien-3-one is a potent competitive progesterone receptor antagonist creating alternatives for the treatment of gynaecological diseases. Amenorrhea was observed in healthy subjects treated with said compound. (11β,17β)-17-Hydroxy-11-[4-(methylsulphonyl)phenyl]-17-(pentafluoroethyl)estra-4,9-dien-3-one was originally disclosed in WO2011/009531A1. Further, it was surprisingly found that the dosage of about 0.5 to 5 mg and more particularly 2 mg of (11β,17β)-17-Hydroxy-11-[4-(methylsulphonyl)phenyl]-17-(pentafluoroethyl)estra-4,9-dien-3-one that is a potent progesterone receptor antagonist being useful for the treatment of certain gynaecological diseases wherein the gynaecological disease is preferably characterized by excessive uterine bleeding. Indeed, it was observed an amenorrhea (non-bleeding) of 92.5% in the healthy subjects to which said compound was administered at a dosage of 2 mg. Amenorrhea corresponds to the major objective of the treatment i.e. control of excessive uterine bleeding.
In the case of the present invention further improvements were observed in respect of the return of bleeding after the end of treatment and the endometrium thickness.

### Summary

The invention is directed to a pharmaceutical composition comprising about 0.5 to 5 mg of (11β,17β)-17-Hydroxy-11-[4-(methylsulphonyl)phenyl]-17-(pentafluoroethyl)estra-4,9-dien-3-one and treatment and/or prophylaxis of gynaecological diseases in patients in need wherein the gynaecological disease is preferably characterized by excessive uterine bleeding.
The invention is directed to a pharmaceutical composition comprising about 2 mg of (11β,17β)-17-Hydroxy-11-[4-(methylsulphonyl)phenyl]-17-(pentafluoroethyl)estra-4,9-dien-3-one and treatment and/or prophylaxis of gynaecological diseases in patients in need wherein the gynaecological disease is preferably characterized by excessive uterine bleeding.
Further, the invention is directed to an oral dosage form comprising about 2 mg of (11β,17β)-17-Hydroxy-11-[4-(methylsulphonyl)phenyl]-17-(pentafluoroethyl)estra-4,9-dien-3-one and treatment and/or prophylaxis of gynaecological diseases in patients in need wherein the gynaecological disease is preferably characterized by excessive uterine bleeding.
Finally, the invention is directed to a method for obtaining said pharmaceutical composition or oral dosage form.
The compound of the invention is defined as a selective progesterone receptor modulator with well confirmed antagonist property.

### Description

In a **first aspect,** the invention is directed to a pharmaceutical composition comprising about 0.5 to 5 mg of (11β, 17β)-17-Hydroxy-11-[4-(methylsulphonyl)phenyl]-17-(pentafluoroethyl)estra-4,9-dien-3-one of formula 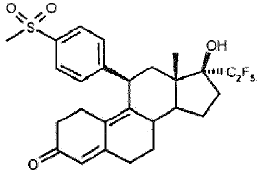 or salts thereof.

Preferably, the pharmaceutical composition comprises a range of about 0.7 to 5 mg, 0.7 to 4.5 mg, 1 to 4.5 mg, 1 to 4 mg, 1.5 to 3.5 mg or 1.5 to 3 mg of above mentioned compound or salt thereof independently from each other. More preferably, the pharmaceutical composition comprises a range of about 0.7 to 5 mg, 1 to 4 mg or 1.5 to 3 mg of above mentioned compound or salt thereof independently from each other. Even more preferably, the pharmaceutical composition comprises a range of about 1 to 4 mg of above mentioned compound or salt thereof.

Preferably, the pharmaceutical composition comprises 0.5 mg, 0.7 mg, 1 mg, 2 mg, 3 mg, 4 mg, or 5 mg of above mentioned compound or salt thereof. More preferably, the pharmaceutical composition comprises 2 mg, 3 mg or 4 mg of above mentioned compound or salt thereof.

Even more preferably, the invention is directed to a pharmaceutical composition comprising about 2 mg of (11β, 17β)-17-Hydroxy-11-[4-(methylsulphonyl)phenyl]-17-(pentafluoroethyl)estra-4,9-dien-3-one of formula 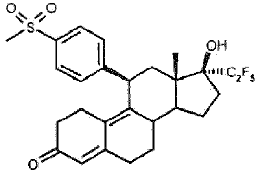 or salts thereof.
In a first embodiment, the pharmaceutical composition comprises additionally a pharmaceutically acceptable excipient.
Pharmaceutically acceptable excipient is defined as a filler (such as sugars, such as lactose, sucrose, dextrose and dextrates; sugar alcohols, such as mannitol, sorbitol and xylitol); carbonates and phosphates of alkaline earth metals, such as calcium carbonate and calcium phosphate; celluloses, such as powdered cellulose and microcrystalline cellulose; colloidal silica; titanium dioxide; kaolin; talc), or lubricants (such as magnesium stearate).

In a second embodiment, the pharmaceutical composition comprises additionally a pharmaceutically acceptable excipient and/or at least one or more other active substances, in particular active substances known for the treatment and/or prophylaxis of the aforementioned diseases.
For the treatment of fibroids of the uterus or endometriosis, the compound according to the invention can be combined simultaneously or sequentially with gestagens or combinations of oestrogens and gestagens.
Progesterone receptor antagonists/gestagen regimens are disclosed in WO 96/15794 (Spicer et al., Balance Pharm. Inc.), WO 96/03130 (Stöckemann et al., Schering AG) and PCT/EP2009/003249 (Moller et al., Bayer Schering Pharma AG). Regimens - optionally repeated - in which the progesterone receptor antagonist is administered over a period of two to four months, followed by the administration of the gestagen for a period of one to four weeks, are very suitable for the treatment of fibroids of the uterus and endometriosis. Administration of the progesterone receptor antagonist for 84 days, followed by administration of the gestagen for 14 days - optionally repeated - is especially suitable.
Simultaneous or sequential administration of the compounds according to the invention e.g. with SERMs, SERDs and oestrogens can be considered for the treatment of complaints associated with the menopause. SERMs (selective estrogen receptor modulators) are compounds that are tissue selective and have either an anti-oestrogenic or oestrogenic action, for example on the uterus they inhibit the action of oestrogen, but on bone they have a neutral or oestrogen-like action. Examples are clomifene, raloxifene, tamoxifen, torimifene, bazedoxifene, lasofoxifene and ormeloxifene.
Selective estrogen receptor destabilizers (SERD) are pharmaceuticals which completely antagonize the oestrogen receptor ('pure anti-oestrogens' without oestrogenic active component) and lead to down-regulation of the receptor (for example fulvestrant, ZK-703 and ZK-253 (Hoffmann J et al., J Natl Cancer Inst 2004, 96:210-218) and compounds described in WO 98/007740, WO 99/33855 and WO 03/045972. Anti-oestrogens are compounds that completely antagonize the oestrogen receptor, for example fulvestrant.

Gestagens are, in the sense of the present invention, either the natural progesterone itself or synthetic derivatives, which like progesterone itself bind to the progesterone receptor and, at dosages above the ovulation inhibiting dose, inhibit ovulation. As examples of the synthetic derivatives, we may mention drospirenone, gestodene, levonorgestrel, cyproterone acetate, desogestrel and 3-ketodesogestrel, norethisterone, norethisterone acetate and dienogest.

Combinations of gestagens and oestrogens are the combinations of active substances that are contained in the oral contraceptives that are known *per se,* for example Yasmin, Femovan, Triquilar, Marvelon, YAZ etc.

The invention encompasses all salts, solvates or solvates of the salts, including all crystal modifications of (11β,17β)-17-Hydroxy-11-[4-(methylsulphonyl)phenyl]-17-(pentafluoroethyl)estra-4,9-dien-3-one.

The pharmaceutical composition is in an appropriate form for intravenous (i.v.), intramuscular (i.m.) or oral administration. Preferably, oral form for administration is a dosage form such as tablet capsule or solution.
Nevertheless, it may optionally be necessary to deviate from the stated amounts, namely depending on body weight, route of administration, individual response to the active substance, type of preparation and point of time or interval when application takes place. Thus, in some cases it may be sufficient to use less than the aforementioned minimum amount, whereas in other cases the stated upper limit must be exceeded. In the case of the administration of larger amounts it may be advisable to distribute these in several individual doses throughout the day.

(11β,17β)-17-Hydroxy-11-[4-(methylsulphonyl)phenyl]-17-(pentafluoroethyl)estra-4,9-dien-3-one is identified as the invention compound and referenced as compound 1 within the whole specification.
It shall be understood that the dosage "about 2 mg" means any dosage from 1.5 to 2.5 mg of compound 1. Preferably, the dosage is of 2 mg of compound 1.

The compound 1 according to the invention displays an unforeseeable, valuable pharmacological, pharmacokinetic and pharmacodynamic profile of action.

In a **second aspect,** the invention is directed to a pharmaceutical composition as described in first aspect useful for the treatment and/or prophylaxis of gynaecological diseases. The gynaecological disease is preferably characterized by excessive uterine bleeding. More preferably, the gynaecological disease is fibroids of the uterus (myomas, uterine leiomyoma), endometriosis or excessive menstrual bleeds. Even more preferably, the gynaecological disease is fibroids of the uterus (myomas, uterine leiomyoma).

In other word the invention is directed to a method for the treatment and/or prophylaxis of gynaecological diseases with the administration to a patient in need of a pharmaceutical composition comprising about 0.5 to 5 mg of (11β,17β)-17-Hydroxy-11-[4-(methylsulphonyl)phenyl]-17-(pentafluoroethyl)estra-4,9-dien-3-one or salts thereof. The gynaecological disease is preferably characterized by excessive uterine bleeding. More preferably, the gynaecological disease is fibroids of the uterus (myomas, uterine leiomyoma), endometriosis or excessive menstrual bleeds. Even more preferably, the gynaecological disease is fibroids of the uterus (myomas, uterine leiomyoma).

Preferably, the pharmaceutical composition comprises a range of about 0.7 to 5 mg, 0.7 to 4.5 mg, 1 to 4.5 mg, 1 to 4 mg, 1.5 to 3.5 mg or 1.5 to 3 mg of above mentioned compound or salt thereof independently from each other. More preferably, the pharmaceutical composition comprises a range of about about 0.7 to 5 mg, 1 to 4 mg or 1.5 to 3 mg of above mentioned compound or salt thereof independently from each other. Even more preferably, the pharmaceutical composition comprises a range of about 1 to 4 mg of above mentioned compound or salt thereof.

Preferably, the pharmaceutical composition comprises 0.5 mg, 0.7 mg, 1 mg, 2 mg, 3 mg, 4 mg, or 5 mg of above mentioned compound or salt thereof. More preferably, the pharmaceutical composition comprises 2 mg, 3 mg or 4 mg of above mentioned compound or salt thereof.

More particularly, the invention is directed to a method for the treatment and/or prophylaxis of gynaecological diseases with the administration to a patient in need of a pharmaceutical composition comprising about 2 mg of (11β,17β)-17-Hydroxy-11-[4-(methylsulphonyl)phenyl]-17-(pentafluoroethyl)estra-4,9-dien-3-one or salts thereof. The gynaecological disease is preferably characterized by excessive uterine bleeding. More preferably, the gynaecological disease is fibroids of the uterus (myomas, uterine leiomyoma), endometriosis or excessive menstrual bleeds. Even more preferably, the gynaecological disease is fibroids of the uterus (myomas, uterine leiomyoma).

In a **third aspect,** the invention is directed to an oral dosage form comprising about 0.5 to 5 mg of (11β,17β)-17-Hydroxy-11-[4-(methylsulphonyl)phenyl]-17-(pentafluoroethyl)estra-4,9-dien-3-one of formula 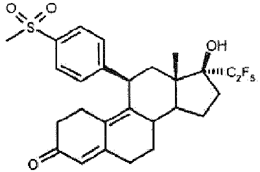 or salts thereof.

Preferably, the pharmaceutical composition comprises a range of about 0.7 to 5 mg, 0.7 to 4.5 mg, 1 to 4.5 mg, 1 to 4 mg, 1.5 to 3.5 mg or 1.5 to 3 mg of above mentioned compound or salt thereof independently from each other. More preferably, the pharmaceutical composition comprises a range of about 0.7 to 5 mg, 1 to 4 mg or 1.5 to 3 mg of above mentioned compound or salt thereof independently from each other. Even more preferably, the pharmaceutical composition comprises a range of about 1 to 4 mg of above mentioned compound or salt thereof.

Preferably, the pharmaceutical composition comprises 0.5 mg, 0.7 mg, 1 mg, 2 mg, 3 mg, 4 mg, or 5 mg of above mentioned compound or salt thereof. More preferably, the pharmaceutical composition comprises 2 mg, 3 mg or 4 mg of above mentioned compound or salt thereof.

More particularly, the invention is directed to an oral dosage form comprising about 2 mg of (11β,17β)-17-Hydroxy-11-[4-(methylsulphonyl)phenyl]-17-(pentafluoroethyl)estra-4,9-dien-3-one of formula 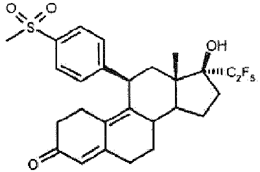 or salts thereof.

In a first embodiment, the oral dosage form comprises additionally a pharmaceutically acceptable excipient.

In a second embodiment, the oral dosage form comprises additionally a pharmaceutically acceptable excipient and/or at least one or more other active substances, in particular active substances known for the treatment and/or prophylaxis of the aforementioned diseases.

Embodiment and preferred features as described above are herein included.

In a **fourth aspect,** the invention is directed to an oral dosage form as described in third aspect for the treatment and/or prophylaxis of gynaecological diseases. The gynaecological disease is preferably characterized by excessive uterine bleeding. More preferably, the gynaecological disease is fibroids of the uterus (myomas, uterine leiomyoma), endometriosis or excessive menstrual bleeds. Even more preferably, the gynaecological disease is fibroids of the uterus (myomas, uterine leiomyoma).

Embodiment and preferred features as described above are herein included.

In a **fifth aspect**, the invention is directed to a method for obtaining said pharmaceutical composition or oral dosage form as described above.

Embodiment and preferred features as described above are herein included.

### Definitions and Preferred features applicable to first to fourth aspect:

Physiologically harmless salts of the compounds according to the invention are preferred as salts within the scope of the present invention. However, salts that are not suitable in themselves for pharmaceutical uses, but can for example be used for the isolation or purification of the compounds according to the invention, are also covered. Physiologically harmless salts of the compounds according to the invention comprise -when they contain a basic function - salts with inorganic or organic acids, in particular of mineral acids, carboxylic acids and sulphonic acids, e.g. salts of hydrochloric acid, hydrobromic acid, sulphuric acid, phosphoric acid, methanesulphonic acid, ethanesulphonic acid, toluenesulphonic acid, benzenesulphonic acid, naphthalene-disulphonic acid, acetic acid, trifluoroacetic acid, propionic acid, lactic acid, tartaric acid, malic acid, citric acid, fumaric acid, maleic acid and benzoic acid.
Physiologically harmless salts of the compounds according to the invention comprise -when they contain an acid function - alkali metal salts, alkaline earth metal salts or ammonium salts, such as can be obtained by reaction with corresponding inorganic or organic bases. We may mention, for example and preferably, alkali metal salts (e.g. sodium and potassium salts), alkaline earth metal salts (e.g. calcium and magnesium salts) and ammonium salts, derived from ammonia or organic amines with 1 to 16 carbon atoms, such as, for example and preferably, ethylamine, diethylamine, triethylamine, ethyldiisopropylamine, monoethanolamine, diethanolamine, triethanolamine, bicyclo-hexylamine, dimethylamino-ethanol, procaine, dibenzylamine, N-methylmorpholine, arginine, lysine, ethylenediamine, N-methyl piperidine, N-methyl glucamine, D-methyl glucamine, ethyl glucamine, 1,6-hexadiamine, glucosamine, N-methylglycine, 2-amino-1,3-propandiol, tris-hydroxymethyl-aminomethane and 1-amino-2,3,4-butanetriol.
Those forms of the compounds according to the invention that display, in the solid or liquid state, adduct formation with solvent molecules, are designated as solvates within the scope of the invention. The solvent can be present in stoichiometric or even non-stoichiometric proportions. In the case of stoichiometric solvates, they are also called hemi-, (semi-), mono-, sesqui-, di-, tri-, tetra-, penta-, etc. solvates. Hydrates are a special form of solvates, in which the coordination takes place with water.

The pharmaceutical efficacy of the compound according to the invention can be explained by their action as progesterone receptor antagonists, and thus by their antagonizing action on the progesterone receptor.
Another object of the present invention is the use of the compound according to the invention for the treatment and/or prophylaxis of diseases based on hormone-dependent hyperproliferative processes, preferably of gynaecological diseases, in particular of fibroids of the uterus, endometriosis or hormone-dependent breast cancers.

The compounds according to the invention can act systemically and/or locally. For this purpose they can be applied in a suitable way, e.g. by the oral, intrauterine, intravaginal, parenteral, pulmonary, nasal, sublingual, lingual, buccal, rectal, dermal, transdermal, conjunctival, or otic route or as an implant or stent. Intrauterine means in particular application by means of an IUS (intrauterine system) or IUD (intrauterine device). Intravaginal application can be effected by means of, among others, IVR/VRS (intravaginal ring/vaginal ring system).
Forms for intrauterine or intravaginal application (cf. e.g. WO 01/47490, especially page 1, line 10 to line 5, line 13 and line 7, line 19 to line 58, line 6, or for vaginal rings: WO 06/010097, especially page 10, line 22 to page 14, line 28) can contain the compounds according to the invention and non-silicone and/or silicone polymers, in particular also siloxane-based elastomers (cf. WO 01/47490, especially page 7, line 19 - page 15, line 15).
For these routes of administration, the compounds according to the invention can be administered in suitable dosage forms.
Quick-release and/or modified-release dosage forms functioning according to the prior art are suitable for oral administration, containing the compounds according to the invention in crystalline and/or amorphous and/or dissolved form, e.g. tablets (uncoated or coated tablets, for example with enteric coatings or delayed-dissolving or insoluble coatings, which control the release of the compound according to the invention), tablets or films/wafers that quickly disintegrate in the oral cavity, films/lyophilizates, capsules (for example hard-gelatin or soft-gelatin capsules), coated tablets, granules, pellets, powders, emulsions, suspensions, aerosols or solutions.
Parenteral application can take place while avoiding an absorption step (e.g. intravenous, intraarterial, intracardial, intraspinal or intralumbar) or with inclusion of absorption (e.g. intramuscular, subcutaneous, intradermal, percutaneous or intraperitoneal). Injection and infusion preparations in the form of solutions, suspensions, emulsions, lyophilizates or sterile powders, among others, are suitable as dosage forms for parenteral administration.
For the other routes of administration, the following are suitable, e.g. inhalation dosage forms (including powder inhalers, nebulizers), nasal drops, solutions, and sprays; tablets for lingual, sublingual or buccal administration, films/wafers or capsules, suppositories, ear or eye preparations, vaginal capsules, aqueous suspensions (lotions, shaking mixtures), lipophilic suspensions, ointments, creams, transdermal therapeutic systems (for example patches), milk, pastes, foams, dusting powders, implants or stents.

The compounds according to the invention can be converted to the aforementioned dosage forms. This can be carried out in a manner that is known *per se,* by mixing with inert, non-toxic, pharmaceutically suitable excipients. These excipients include, among others, carrier substances (for example microcrystalline cellulose, lactose, mannitol), solvents (e.g. liquid polyethylene glycols), emulsifiers and dispersants or wetting agents (for example sodium dodecylsulphate, polyoxysorbitan oleate), binders (for example polyvinylpyrrolidone), synthetic and natural polymers (for example albumin), stabilizers (e.g. antioxidants, for example ascorbic acid), colouring matter (e.g. inorganic pigments, for example iron oxides) and taste and/or odour correctants.

### Experimental part

The percentages in the following tests and examples are, unless stated otherwise, percentages by weight; parts are parts by weight. Proportions of solvents, dilution ratios and concentration figures for liquid/liquid solutions always refer to volume.

The following examples serve to explain the invention without limiting it in any way.

### Example 1: Synthesis path of compound 1

### (11β,17β)-17-Hydroxy-11-[4-(methylsulphonyl)phenyl]-17-(pentafluoroethyl)estra-4,9-dien-3-one

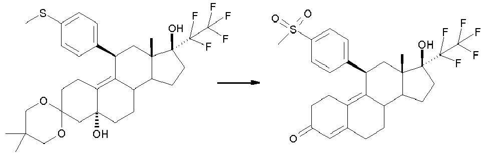

5g of the compound described in example 1b) was dissolved in a mixture of 140 ml THF and 140 ml methanol. A solution of 20 g Oxone® in 94 ml water was slowly added dropwise at 0°C. Then it was stirred for a further 3.5 hours at 0°C. Then a mixture of water and dichloromethane was added to the reaction mixture. The phases were separated and the aqueous phase was extracted several times with dichloromethane. The combined organic phases were washed with saturated aqueous sodium chloride solution, dried over sodium sulphate and concentrated under vacuum. The raw product was purified by silica gel chromatography. This gave 3.8 g of the title compound.
¹H-NMR (300 MHz, CDCl₃): δ= 7.86 d (2H); 7.40 d (2H); 5.81 sbr (1H); 4.50 dbr (1H); 3.07 s (3H); 0.51 s (3H).

### Example 2: Dose-effect of 84 days treatment with compound 1

### Randomized Study population:

1. Healthy female subjects, sterilized by tubal ligation
2. Age at screening: 18 - 45 years
3. Body mass index (BMI) at screening: ≥ 18 and ≤ 32 kg/m²
4. At least 3 consecutive regular menstrual cycles with a cycle length of 24 - 35 days before first screening examination according to the subject's history
5. Absence of clinically relevant abnormal findings in the pre-treatment endometrial biopsy
6. Adequate venous access (frequent blood sampling)

### Protocol:

**Table 1: Treatment dosage with compound 1**

| Dose level | Treatment (Dose **compound 1**) for 84 days | Amount / route of administration | Cumulative dose per subject | | No. of subjects treated |
|---|---|---|---|---|---|
| A | 0.1 mg | 1 tablet 0.1 mg **compound 1** and 3 tablets placebo once daily per os | 8.4 mg | | 10 |
| | | | | | |
| B | 0.5 mg | 1 tablet 0.5 mg **compound 1** and 3 tablets placebo once daily per os | 42 mg | | 10 |
| | | | | | |
| C | 1 mg | 2 tablets 0.5 mg **compound 1** and 2 tablets placebo once daily per os | 84 mg | | 10 |
| | | | | | |
| D | 2 mg | 4 tablets 0.5 mg **compound 1** once daily per os | 168 mg | | 10 |
| | | | | | |
| E | 5 mg | 1 tablet 5 mg **compound 1** and 3 tablets placebo once daily per os | 420 mg | | 10 |
| | | | | | |
| P | Placebo | 4 tablets placebo once daily per os | 0 mg | | 10 |

The subject had to start the intake of the study drugs on the first or second day of menstrual bleeding after the pre-treatment cycle. The pre-treatment cycle started on the first day of the subject's menstrual bleeding after screening examinations had shown that the subject was eligible for further participation. After the pre-treatment cycle treatment was started. The treatment period started with the first and ended with the last intake of compound 1. For each intake day the subject received one bottle containing 4 tablets. The number of tablets taken and the intake time had to be documented in the diary.

The evaluation of bleeding pattern was based on a daily self-assessment of the bleeding intensity by the subject. These assessments were categorized as defined below. The subjects were provided with an explanation of the categories in local language, and were asked to document the bleeding intensity in their diaries accordingly (one entry per day).
Administration of compound 1 occurred during 84 days (multiple doses administration).

**Table 2: Bleeding pattern categories**

| **Code** | **Category** | **Definition** |
|---|---|---|
| 1 | None | No bleeding |
| 2 | Spotting | Less than associated with normal menstruation relative to the subject's experience, with no need for sanitary protection (except for panty liners) |
| 3 | Light | Less than associated with normal menstruation relative to the subject's experience, with need for sanitary protection |
| 4 | Normal | Like normal menstruation relative to the subject's experience |
| 5 | Heavy | More than normal menstruation relative to the subject's experience |

### Results:

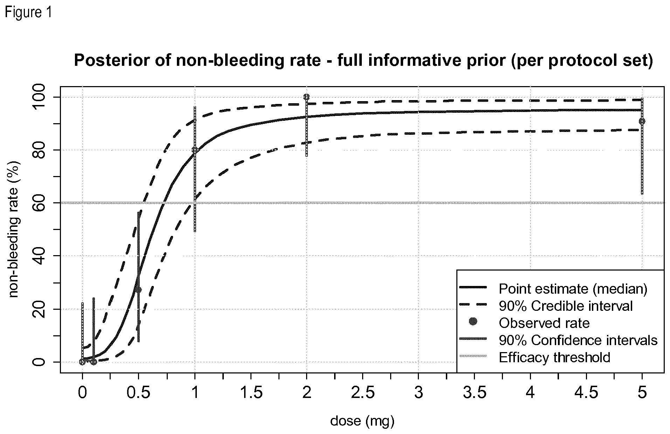

Results of the bleeding pattern is shown in figure 1 (posterior of non-blrrding (per protocol set).
It was observed that compound 1 caused a marked and dose-dependent reduction in the number of days of bleeding during the treatment period. After administration of 0.5 mg of compound 1, three (3) out of eleven (11) subjects (27 %) had no further bleeding during the treatment period of 84 days. 50% of dose-dependent reduction of bleeding is obseverd with a dosage of 0.7 mg. Clear stagnation appears around 2 mg and from 2 mg up to 5 mg a saturation is observed around 95%.

### Example 3: Return of bleeding after end of treatment (84 days) with compound 1 Randomized Study population:

As described in example 2.

### Protocol:

The first day of bleeding after the end of the treatment was marked.

### Results

Subjects of the study treated with 2 mg of compound 1 during 84 days show a delayed return of bleeding with a mean of 25 days. A delayed return of bleeding after treatment leads to longer free bleeding periods per year for the subject. One subject showed a delayed return of bleeding after treatment of about 52 days. All results are in table 3 below.

**Table 3: Number of days until onset of bleeding after end of treatment (mean ± SD, min - max; PPS, n = 67).**

| | Plac (n = 12) | 0.1 mg (n = 11) | 0.5 mg (n = 11) | 1 mg (n = 10) | 2 mg (n = 12) | 5 mg (n = 11) |
|---|---|---|---|---|---|---|
| Number of days until first bleeding after end of treatment | 9.8 ± 8 (1 - 23) | 12.2 ± 9.9 (3 - 35) | 14.8 ± 10.2 (1 - 28) | 20.3 ± 6.2 (8 - 28) | 25.8 ± 11.1 (9 - 52) | 20.9 ± 10.9 (1 - 38) |

| | | | | | | |
|---|---|---|---|---|---|---|
| Plac: Placebo | | | | | | |

### Example 4: Measurement of endometrial thickness during and after treatment with compound 1 Randomized Study population

As described in example 2.

### Protocol:

The endometrial thickness was measured in the medio-sagittal section as double-layer in millimeters using transvaginal ultrasound.

### Results:

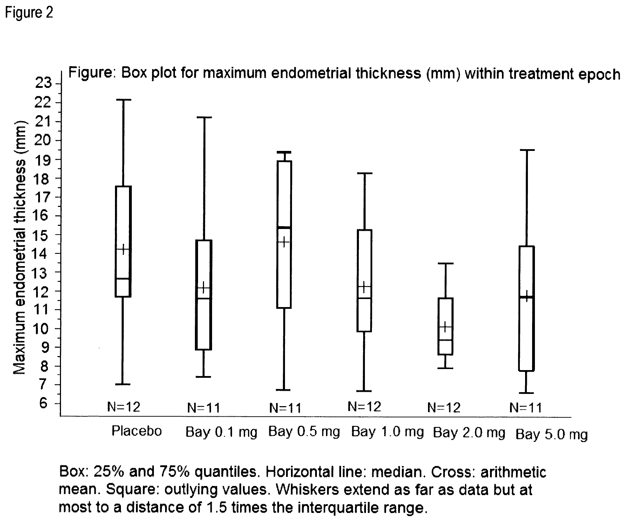

Subjects of the study treated with 2 mg of compound 1 during 84 days show a lower maximum thickness of the endometrium compared to other dosages of compound 1. Further, the observed results of the subjects of said group are more consistent than other dosage groups.
See figure 2 (Box plot for maximum endometrial thickness within treatment epoch).

Bay means compound 1.

### Example 5: Ovulation inhibition: Follicle size, Estradiol (E2), Progesterone (P)

**Protocol:** Randomized Study population of healthy female subjects as described in example 2 above. Blood samples were obtained from healthy female subjects for the determination of Estradiol (E2), Progesterone (P), luteinizing hormone (LH) and follicle stimulating hormone (FSH) in serum and taken at the same time points as listed below during the pre-treatment cycle, the treatment and follow-up cycle. Transvaginal ultrasound (TVU) was used to monitor ovarian follicular growth during pretreatment (days 9 and 21), treatment (days 7, 14, 21, 28, 35, 42, 49, 56, 63, 70, 77, 84) and follow-up cycle (days 9 and 21).

### Results:

Table 4 shows the number and percentage of subjects, classified according to the maximum follicle size and Estradiol (E2) and Progesterone (P) values during the treatment period (number of subjects n = 69)

During treatment, maximum diameters of Follicle-like structures (FLS) were between 13 and 30 mm in the majority of subjects of all treatment groups (including placebo).

No ovulation occurred in most of the subjects receiving dosages ≥ 0.5 mg of compound 1 during subject treatment of 84 days (i.e. progesterone value < 1.57 µg/L).

**Table 4**

| | **Placebo (n = 12)** | **0.1 mg (n = 11)** | **0.5 mg (n = 11)** | **1 mg (n = 12)** | **2 mg (n = 12)** | **5 mg (n=11)** |
|---|---|---|---|---|---|---|
| Follicle size ≤ 13 mm | 2 (16 %) | 1 (9 %) | - | 1 (8 %) | - | 1 (9 %) |
| Follicle size > 13 mm + Estradiol > 27.2 pg/mL + Progesterone < 1.57 µg/L | - | - | 6 (55 %) | 9 (75 %) | 11 (92 %) | 9 (82 %) |
| Follicle size > 13 mm + Estradiol > 27.2 pg/mL + Progesterone > 1.57 µg/L | 10 (83 %) | 10 (91 %) | 5 (45 %) | 2 (17%) | 1 (8 %) | 1 (9 %) |

## Claims

1. A pharmaceutical composition comprising about 1 to 5 mg of (11β,17β)-17-Hydroxy-11-[4-(methylsulphonyl)phenyl]-17 -(pentafluoroethyl)estra-4, 9-dien-3-one of formula 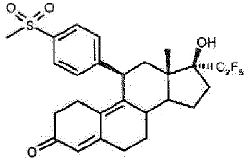 or salts thereof to be administered in patients in need for use in the treatment or prophylaxis of a gynaecological disease.

2. The pharmaceutical composition for use according to claim 1 wherein the pharmaceutical composition comprises about 1 to 4 mg of (11β,17β)-17-Hydroxy-11-[4-(methylsulphonyl)phenyl]-17-(pentafluoroethyl)estra-4,9-dien-3-one.

3. The pharmaceutical composition for use according to claim 1 to 2 wherein the pharmaceutical composition comprises about 2 mg of (11β,17β)-17-Hydroxy-11-[4-(methylsulphonyl)phenyl]-17-(pentafluoroethyl)estra4,9-dien-3-one.

4. The pharmaceutical composition for use according to claim 1, 2 or 3 comprising additionally a pharmaceutically acceptable carrier.

5. The pharmaceutical composition or use according to claims 1 to 4 in a form of an oral dosage, wherein the dosage form is a tablet or a capsule.

6. The pharmaceutical composition according to claims 1 to 5 for use in the treatment and/or prophylaxis of gynaecological disease that is preferably **characterized by** excessive uterine bleeding, such as fibroids of the uterus (myomas, uterine leiomyoma), endometriosis or excessive menstrual bleeds.

7. The pharmaceutical composition according to claim 6 for use in the treatment of fibroids of the uterus (myomas, uterine leiomyoma).

8. Oral dosage form comprising about 1 to 5 mg of (11β,17β)-17-Hydroxy-11-[4-(methylsulphonyl)phenyl]-17 -(pentafluoroethyl)estra-4, 9-dien-3-one of formula 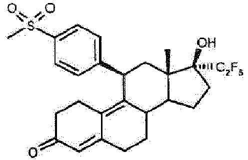 or salts thereof.

9. The Oral dosage form according to claim 8 comprising about 2 mg of (11β,17β)-17-Hydroxy-11-[4-o(methylsulphonyl)phenyl]-17 -(pentafluoroethyl)estra-4, 9-dien-3-one of formula 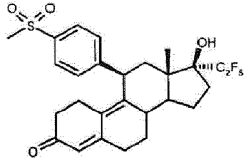 or salts thereof.

10. The oral dosage form according to claim 8 or 9 for the treatment and/or prophylaxis of gynaecological disease that is preferably **characterized by** excessive uterine bleeding, such as fibroids of the uterus (myomas, uterine leiomyoma), endometriosis or excessive menstrual bleeds.

11. The oral dosage form according to claim 10 for the treatment of fibroids of the uterus (myomas, uterine leiomyoma).

12. A method for obtaining a pharmaceutical composition as described in claims 1 to 7 by mixing 1 to 5 mg of (11β,17β)-17-Hydroxy-11-[4-(methylsulphonyl)phenyl]-17-(pentafluoroethyl)estra-4,9-dien-3-one of formula 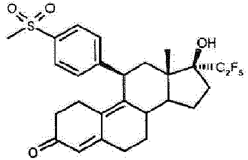 with inert, non-toxic, pharmaceutically suitable excipient.

13. A method for obtaining an oral dosage form as described in claims 8 to 11 by mixing 1 to 5 mg of (11β,17β)-17-Hydroxy-11-[4-(methylsulphonyl)phenyl]-17-(pentafluoroethyl)estra-4,9-dien-3-one of formula 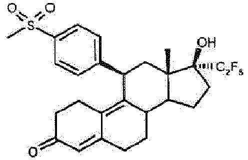 with inert, non-toxic, pharmaceutically suitable excipient where the oral dosage form is a tablet or a capsule.

## Patentansprüche

1. Pharmazeutische Zusammensetzung, umfassend etwa 1 bis 5 mg (11β,17β)-17-Hydroxy-11-[4-(methylsulfonyl)phenyl]-17-(pentafluorethyl)estra-4,9-dien-3-on der Formel 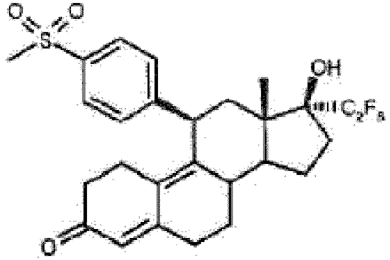 oder Salze davon, zur Verabreichung an bedürftige Patienten zur Verwendung bei der Behandlung oder Prophylaxe einer gynäkologischen Krankheit.

2. Pharmazeutische Zusammensetzung zur Verwendung nach Anspruch 1, wobei die pharmazeutische Zusammensetzung etwa 1 bis 4 mg (11β,17β)-17-Hydroxy-11-[4-(methylsulfonyl)phenyl]-17-(pentafluorethyl)estra-4,9-dien-3-on umfasst.

3. Pharmazeutische Zusammensetzung zur Verwendung nach Anspruch 1 bis 2, wobei die pharmazeutische Zusammensetzung etwa 2 mg (11β,17β)-17-Hydroxy-11-[4-(methylsulfonyl)phenyl]-17-(pentafluorethyl)-estra-4,9-dien-3-on umfasst.

4. Pharmazeutische Zusammensetzung zur Verwendung nach Anspruch 1, 2 oder 3, welche zusätzlich einen pharmazeutisch unbedenklichen Träger umfasst.

5. Pharmazeutische Zusammensetzung zur Verwendung nach Ansprüchen 1 bis 4 in Form einer oralen Dosierung, wobei es sich bei der Dosierungsform um eine Tablette oder eine Kapsel handelt.

6. Pharmazeutische Zusammensetzung zur Verwendung nach Ansprüchen 1 bis 5 zur Verwendung bei der Behandlung und/oder Prophylaxe einer gynäkologischen Krankheit, die vorzugsweise durch exzessive Uterusblutung gekennzeichnet ist, wie Uterusfibroiden (Myomen, Uterusleiomyom), Endometriose oder exzessive Menstruationsblutungen.

7. Pharmazeutische Zusammensetzung nach Anspruch 6 zur Verwendung bei der Behandlung von Uterus fibroiden (Myomen, Uterusleiomyom).

8. Orale Dosierungsform, umfassend etwa 1 bis 5 mg an (11β, 17β)-17-Hydroxy-11-[4-(methylsulfonyl)-phenyl]-17-(pentafluorethyl)estra-4,9-dien-3-on der Formel 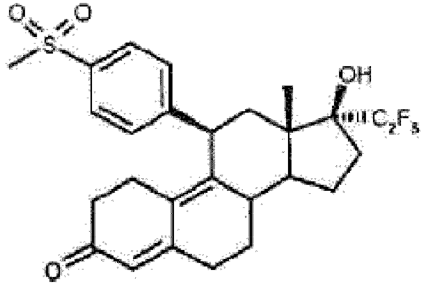 oder Salzen davon.

9. Orale Dosierungsform nach Anspruch 8, umfassend etwa 2 mg an (11β,17β)-17-Hydroxy-11-[4-(methylsulfonyl)phenyl]-17-(pentafluorethyl)estra-4,9-dien-3-on der Formel 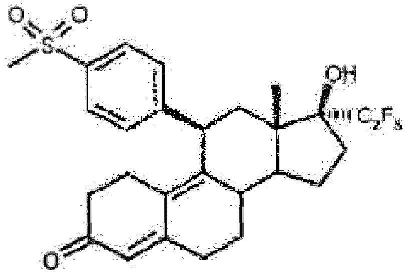 oder Salzen davon.

10. Orale Dosierungsform nach Anspruch 8 oder 9 zur Behandlung und/oder Prophylaxe einer gynäkologischen Krankheit, die vorzugsweise durch exzessive Uterusblutung gekennzeichnet ist, wie Uterusfibroiden (Myomen, Uterusleiomyom), Endometriose oder exzessive Menstruationsblutungen.

11. Orale Dosierungsform nach Anspruch 10 zur Behandlung von Uterus fibroiden (Myomen, Uterus leiomyom).

12. Verfahren zum Erhalt einer wie in den Ansprüchen 1 bis 7 beschriebenen pharmazeutischen Zusammensetzung durch Mischen von 1 bis 5 mg (11β,17β)-17-Hydroxy-11-[4-(methylsulfonyl)phenyl]-17-(pentafluorethyl)estra-4,9-dien-3-on der Formel 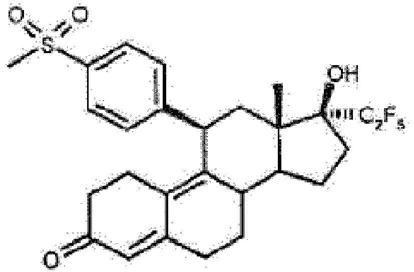 mit einem inerten nichttoxischen, pharmazeutisch geeigneten Exzipienten.

13. Verfahren zum Erhalt einer wie in den Ansprüchen 8 bis 11 beschriebenen oralen Dosierungsform durch Mischen von 1 bis 5 mg (11β,17β)-17-Hydroxy-11-[4-(methylsulfonyl)phenyl]-17-(pentafluorethyl)estra-4,9-dien-3-on der Formel 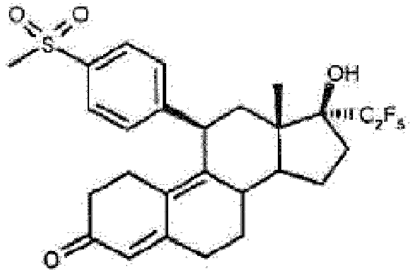 mit einem inerten nichttoxischen, pharmazeutisch geeigneten Exzipienten, wobei es sich bei der oralen Dosierungsform um eine Tablette oder eine Kapsel handelt.

## Revendications

1. Composition pharmaceutique comprenant entre environ 1 et 5 mg de (11β,17β)-17-Hydroxy-11-[4-(méthylsulfonyl)phényl]-17-(pentafluoroéthyl)-oestra-4,9-dién-3-one de formule 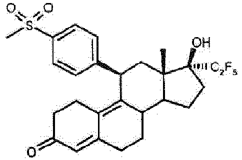 ou les sels de celle-ci pour administration à des patients le nécessitant pour utilisation dans le traitement prophylactique ou thérapeutique d'une maladie gynécologique.

2. Composition pharmaceutique pour utilisation selon la revendication 1 où la composition pharmaceutique comprend entre environ 1 et 4 mg de (11β,17β)-17-Hydroxy-11-[4-(méthylsulfonyl)phényl]-17-(pentafluoroéthyl)-oestra-4, 9-dién-3-one.

3. Composition pharmaceutique pour utilisation selon la revendication 1 à 2 où la composition pharmaceutique comprend environ 2 mg de (11β,17β)-17-Hydroxy-11-[4-(méthylsulfonyl)phényl]-17-(pentafluoroéthyl)-oestra-4,9-dién-3-one.

4. Composition pharmaceutique pour utilisation selon la revendication 1, 2 ou 3 comprenant de plus un véhicule pharmaceutiquement acceptable.

5. Composition pharmaceutique pour utilisation selon les revendications 1 à 4 sous une forme galénique orale, où la forme galénique est un comprimé ou une capsule.

6. Composition pharmaceutique selon les revendications 1 à 5 pour utilisation dans le traitement prophylactique et/ou thérapeutique d'une maladie gynécologique qui est préférentiellement **caractérisée par** une hémorragie utérine excessive, tels que les fibromes utérins (myomes, léiomyomes utérins), l'endométriose ou les hémorragies menstruelles excessives.

7. Composition pharmaceutique selon la revendication 6 pour utilisation dans le traitement des fibromes utérines (myomes, léiomyomes utérins).

8. Forme galénique orale comprenant entre environ 1 et 5 mg de (11β,17β)-17-Hydroxy-11-[4-(méthylsulfonyl)phényl]-17-(pentafluoroéthyl)-oestra-4,9-dién-3-one de formule 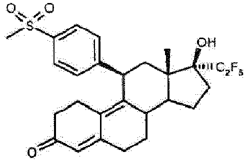 ou les sels de celle-ci.

9. Forme galénique orale selon la revendication 8 comprenant environ 2 mg de (11β,17β)-17-Hydroxy-11-[4-(méthylsulfonyl)phényl]-17-(pentafluoroéthyl)-oestra-4,9-dién-3-one de formule 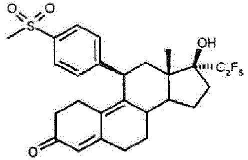 ou les sels de celle-ci.

10. Forme galénique orale selon la revendication 8 ou 9 destinée au traitement prophylactique et/ou thérapeutique d'une maladie gynécologique qui est préférentiellement **caractérisée par** une hémorragie utérine excessive, tels que les fibromes utérines (myomes, léiomyomes utérins), l'endométriose ou les hémorragies menstruelles excessives.

11. Forme galénique orale selon la revendication 10 destinée au traitement des fibromes utérines (myomes, léiomyomes utérins).

12. Procédé d'obtention d'une composition pharmaceutique selon les revendications 1 à 7 par mélangeage d'entre 1 et 5 mg de (11β,17β)-17-Hydroxy-11-[4-(méthylsulfonyl)phényl]-17-(pentafluoroéthyl)-oestra-4,9-dién-3-one de formule 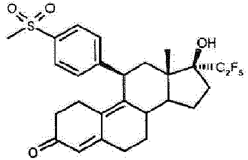 avec un excipient inerte, non toxique, pharmaceutiquement adapté.

13. Procédé d'obtention d'une forme galénique orale selon les revendications 8 à 11 par mélangeage d'entre 1 et 5 mg de (11β,17β)-17-Hydroxy-11-[4-(méthylsulfonyl)phényl]-17-(pentafluoroéthyl)-oestra-4,9-dién-3-one de formule 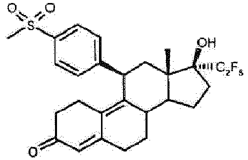 avec un excipient inerte non toxique pharmaceutiquement adapté où la forme galénique orale est un comprimé ou une capsule.
